# EUROPEAN PATENT APPLICATION

(11) **EP 3 335 617 A1**
(43) Date of publication of application: **20.06.2018**
(21) Application number: 16835294.6
(22) Date of filing: 29.06.2016
(51) Int. Cl.: A61B 1/00, A61B 1/06

(54) **ENDOSCOPE**

(30) Priority: 11.08.2015 KR 20150113466
(71) Applicant: Kang, Yoon Sik, Seoul 06718 (KR); Shin, Chung Yoon, Seoul 06718 (KR); Kang, Hyun Suk, Seoul 06718 (KR); Kang, Hyun Yee, Seoul 06718 (KR)
(72) Inventor: Kang, Yoon Sik, Seoul 06718 (KR); Shin, Chung Yoon, Seoul 06718 (KR); Kang, Hyun Suk, Seoul 06718 (KR); Kang, Hyun Yee, Seoul 06718 (KR)
(74) Representative: Gulde & Partner
(86) International application number: PCT/KR2016/006938
(87) International publication number: WO 2017/026653

(57) **Abstract**

Provided is an endoscope capable of more precisely observing curves in the large intestine. The endoscope according to one embodiment of the present disclosure includes a front end part in which a filming device having a fish eye lens is installed; a bending part connected to the front end part and including a plurality of bending elements; an insertion part; and a manipulation part. The insertion part is a part connecting the bending part and the manipulation part and moves the front end part and the being part in a coelom as at least a portion of the insertion part is inserted into or withdrawn from the coelom. The front end part has a projection protruding forward and tapered to have a decreasing width toward its tip. The filming device is installed at the tip of the projection.

## Description

### TECHNICAL FIELD

The present disclosure relates to an endoscope, and more particularly, to an endoscope capable of more precisely observing curves in the large intestine.

### BACKGROUND

In general, the primary purpose of an endoscopic examination of the large intestine is to detect and remove a colon polyp (a part of the tissue protruding from an internal wall of the large intestine, which should be removed upon detection of the colon polyp) that is a prodromal change of a colon, thereby fundamentally preventing the colon cancer from occurring. Despite the above purpose, however, it has been known that a failure ratio of detection of the polyp in the endoscopic examination of the large intestine is approximately up to 30%.

Whether residual stool remains in the large intestine (as a result of insufficient cleaning of the large intestine), a proficiency or sincerity of an examiner, a short examination time and the like are mentioned as causes of a failure of detection of the colon polyp. However, a more fundamental cause of the failure of detection of the polyp results from an anatomical structure of the large intestine.

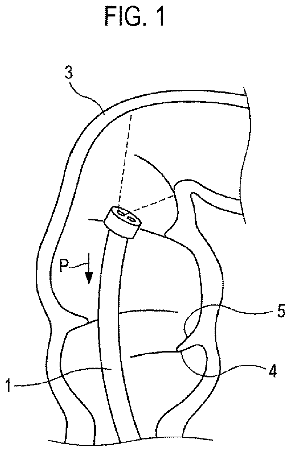

That is, the large intestine 3 is formed with a plurality of curves 4 like the structure of an accordion, as shown in FIG. 1. In the endoscopic examination of the large intestine, after a deep insertion of an endoscope 1 into the large intestine 3, the examiner slowly withdraws the endoscope 1 from the large intestine 3 in a withdrawal direction P while observing the internal wall of the large intestine through a filming device positioned at a front part of the endoscope 1. In such endoscopic examination, however, it is difficult to observe back surfaces 5 of the curves 4 of the internal wall of the large intestine through the filming device provided at the front part of the endoscope 1. Therefore, there is a problem in that it is not easy to detect a polyp formed on the back surfaces 5 of the curves 4.

For this reason, an endoscope of which a bendable angle is increased has been developed to enable the examiner to observe a polyp formed on the back surfaces of the curves by bending the endoscope. However, it is realistically nearly impossible to bend the endoscope at every curve in the large intestine having a small inner diameter for the observation. Meanwhile, an angle of view of a lens of a currently used endoscope for the large intestine is generally 140 degrees, and an endoscope for the large intestine that has a wide-angle lens enabling observation through 170 degrees has been also developed and used in recent years. However, since a lateral side or rear side of the endoscope cannot be observed even through such a wide-angle lens is used, it cannot be believed that a limitation on discovery of a lesion on the back surfaces of the curves has been completely eliminated.

Furthermore, an endoscope in which an additional ring provided with a filming device capable of observing a lateral side of the endoscope is installed at a side surface of a front end part of the endoscope, or an endoscope in which a filming device capable of observing a lateral side of the endoscope is installed at a side surface of a front end part of the endoscope has been developed, although these endoscopes are also unsuitable for actual practice.

In a case where a side lens is mounted on a side surface of an endoscope to enable side observation in addition to a front lens installed at a front part of the endoscope for front observation, mounting only one side lens on the side surface is not sufficient to achieve the side observation and thus at least two side lenses should be mounted on the side surface. Therefore, since a user should simultaneously observe at least three pictures filmed through the respective lenses, it is not possible to exclude a probability that a lesion appearing in the pictures may not be recognized by the user. Moreover, in order to resect a lesion discovered through the side lens or lenses, the lesion should be confirmed with the front lens. However, the process of confirming the lesion, which has been discovered through the side lens or lenses, with the front lens is not intuitive and thus is difficult to perform in many cases.

### SUMMARY

The present disclosure has been conceived to solve the aforementioned problems of the related art, and an object of the present disclosure is to provide an endoscope capable of more precisely observing a hidden lesion in curves in the large intestine and facilitating safe insertion of the endoscope in the large intestine having severe curves.

An endoscope according to one embodiment of the present disclosure incldues a front end part; a bending part connected to the front end part and including a plurality of bending elements; an insertion part; and a manipulation part. The insertion part is a part connecting the bending part and the manipulation part and moves the front end part and the being part in a coelom as at least a portion of the insertion part is inserted into or withdrawn from the coelom. The front end part has a projection protruding forward and tapered to have a decreasing width toward its tip. At least one channel opening is provided in a surface of the projection, at least one light part is provided in a surface of the projection, and a filming device having a fish eye lens is installed at the tip of the projection.

With the use of the fish eye lens instead of an existing planar lens, the range of view that was limited up to 170 degrees can be expanded to 180 degrees or more above 170 degrees, thereby enabling observation of the lateral side and rear side of the endoscope. Accordingly, in case of the large intestine, back surfaces as well as front surfaces of curves of the intestine can also be easily observed, thereby dramatically increasing a discovery rate for a lesion on the back surfaces of the curves, which was difficult to find using an existing endoscope. In addition, it is possible to allow the process of inserting the endoscope through a portion of the intestine bent at an acute angle to be easily and very safely performed, thereby dramatically reducing a risk of enterobrosia.

### BRIFE DESCRIPTION OF THE DRAWINGS

FIG. 1 is a view schematically showing a state in which the large intestine is observed by an endoscope.
FIG. 2 shows an endoscope system according to one embodiment of the present disclosure.
FIG. 3 is a front view of an endoscope according to one embodiment of the present disclosure.
FIG. 4 is an enlarged side view of "A" portion of FIG. 2.

### DETAILED DESCRIPTION

Hereinafter, embodiments of the present disclosure will be described in detail with reference to the accompanying drawings. In the following description, however, detailed descriptions of well-known functions and structures will be omitted.

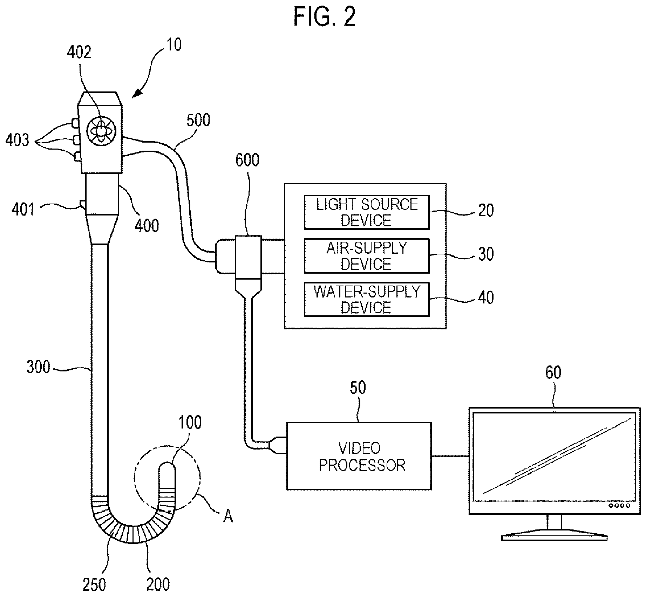

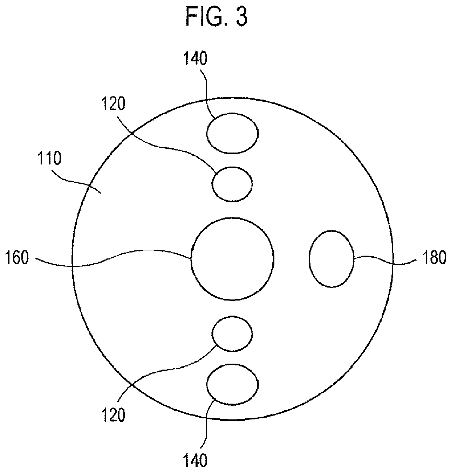

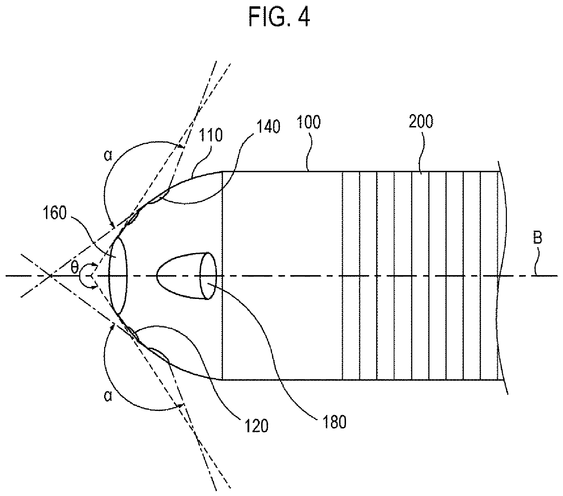

FIG. 2 shows an endoscope system according to one embodiment of the present disclosure. FIG. 3 is a front view of a front end part 100 of an endoscope 10, and FIG. 4 is an enlarged view of "A" portion of FIG. 2.

Referring to FIGS. 2 and 3, the endoscope system may include the endoscope 10, a light source device 20, an air-supply device 30, a water-supply device 40, a video processor 50 and a monitor 60.

The endoscope 10 is a device inserted into a coelom (for example, large intestine) to observe a biological tissue in the coelom and will be described in detail later. The light source device 20 is a device connected to a connector part 600 of the endoscope 10 and controls a light part 140. The video processor 50 is a device connected to the connector part 600 of the endoscope 10, where the video processor receives images filmed by a filming device 160 of the endoscope 10, via the connector part 600 and processes the images. The monitor 60 is connected to the video processor 50 and displays the images processed in the video processor 50. The air-supply device 30 and the water-supply device 40 are connected to the connector part 600 of the endoscope 10 to supply air and water, respectively, which are required for washing the endoscope 10.

The endoscope 10 includes the front end part 100, a bending part 200, an insertion part 300, a manipulation part 400, a universal cord 500 and the connector part 600.

The front end part 100 is a part disposed at a foremost end of the endoscope 10. As shown in FIG. 3, a lens cleaning nozzle 120 for supplying water and air, the light part 140 for radiating light, the filming device 160 for filming the interior of the large intestine, and a channel opening 180 through which forceps or the like can be inserted into the large intestine are provided on a front face of the front end part 100.

The lens cleaning nozzle is connected to the air-supply device 30 and the water-supply device 40 via the universal cord 500 and the connector part 600 by means of a tube (not shown) within the endoscope 10. Accordingly, water or air is sprayed through the lens cleaning nozzle 120 for removing residual stool around the filming device 160 or for washing the filming device 160.

The light part 140 is connected to the light source device 20 via the universal cord 500 and the connector part 600 by means of wiring (not shown) within the endoscope 10. Accordingly, turning-on/off and the like of the light part 140 is controlled by the light source device 20 to irradiate a region to be observed.

The filming device 160 is connected to the video processor 50 via the universal cord 500 and the connector part 600 by means of wiring (not shown) within the endoscope 10. The actuation of the filming device 160 is controlled by the video processor 50 to film the region to be observed, to convert the filmed image into electronic signals and to transmit them to the video processor 50. For example, the filming device 160 may include a lens for functioning as an optical system, a solid state imaging device arranged at an image formation location of the lens to film the region to be observed, a correlated double sampling (CDS) circuit for performing a correlated double sampling process for imaging signals created by the solid state imaging device, and an analog/digital conversion circuit for converting analog imaging signals processed in the CDS circuit into digital imaging signals. The lens is a fish eye lens having an angle of view of 180 degrees or more (i.e., enabling observation through 180 degrees or more).

The channel opening 180 is connected to an instrument channel 401 extending through the interiors of the front end part 100, the bending part 200, the insertion part 300 and the manipulation part 400, and an instrument such as forceps or the like is inserted through the instrument channel 401 into the large intestine through the channel opening 180.

The bending part 200 is connected to a rear side of the front end part 100 and this bending part 200 serves as a part for causing the front end part 100 to be vertically and horizontally directed depending on the shape and position of the region to be observed. The bending part 200 includes a plurality of bending elements 250 connected to each other, and the bending elements 250 are connected to a bendable wire (not shown) passing through the insertion part 300 and connected to an adjustment knob 402 of the manipulation part 400. As an operator rotates the adjustment knob 402, the bendable wire is pulled or unwound, whereby the bending part 200 can be horizontally and vertically bended.

The insertion part 300 is a part connecting the bending part 200 and the manipulation part 400 and is made of a flexible material. As at least a portion of the insertion part 300 is inserted into or withdrawn from the coelom to move the front end part 100 and the bending part 200 in the coelom.

The manipulation part 400 may include the adjustment knob 402 for adjusting a curvature of the bending part 200, and a control valve 403 provided at tubing through which water and air are supplied into an organ that is being examined, to adjust the supply of air or water. The universal cord 500 is a part for connecting the manipulation part 400 and the connector part 600 and includes a wiring and a tube provided therein. The connector part 600 provides interfaces for connecting the wiring and the tube in the universal cord 500 to the light source device 20, the air-supply device 30, the water-supply device 40 and the video processor 50.

FIG. 4 is an enlarged view of "A" portion of FIG. 2 in the endoscope according to the embodiment of the present disclosure. Referring to FIG. 4, the front end part 100 has a projection 110 protruding forward and tapered to have a decreasing width toward its tip. Although FIG. 4 shows that the projection 110 is a portion of the front end part 100, the projection 110 may form the entire front end part 100. The projection 110 may have, for example, a conical structure or a dome structure, so that a cross section of the projection 110 taken in a direction perpendicular to a longitudinal direction of the endoscope is a circle in shape. A longitudinal axis B of the endoscope passes through a center of the circle. The tip of the projection 110 is equipped with the filming device 160. The lens of the filming device is a fish eye lens. The term "fish eye lens" used herein refers to a lens having an angle of view θ of 180 degrees or more (i.e., enabling observation through 180 degrees or more). Thus, the fish eye lens can film the lateral side and rear side as well as the front side of the endoscope on one picture. Since the fish eye lens is mounted at the tip of the projection 110, which is tapered to have a decreasing width toward its tip, rather than installing a filming device at a flat surface of a front end part as in an existing endoscope, an edge of the front end part 100 of the endoscope does not hinder a range of vision of the fish eye lens when the side and rear observation is performed by the fish eye lens.

Although the fish eye lens usually undergoes an image distortion that becomes severer toward a periphery of the lens, this image distortion phenomenon amounts to nothing in performing the observation in the large intestine using the endoscope according to the present disclosure. This is because a region in an image filmed through a front portion of the fish eye lens is subjected to a very small distortion and a region in the image filmed through a side portion of the lens is subjected to a distortion that becomes severer toward the periphery of the lens, although if a lesion is discovered through the side portion of the lens, the bending part 200 can be bent such that the discovered lesion is placed in front of the front end part 100 and a required manipulation such as resection can be then performed.

Additionally, when the angle of view θ of 180 degrees or more is realized, an extending direction of a cavity of the large intestine can be easily checked during insertion of the endoscope, thereby remarkably reducing a risk of enterobrosia when the endoscope passes through a sigmoid-descending colon transition, a bent portion of the spleen or liver, or the like, which has a severe bending of 90 degrees or more with respect to the extending direction. A severest complication in an endoscopic examination of the large intestine is enterobrosia occurring during the insertion of the endoscope, wherein most reasons for the occurrence of enterobrosia are that an existing endoscope having a limited angle of view cannot directly check the extending direction of the intestine turned at an acute angle and thus a force may be unreasonably applied in an incorrect direction. However, when the angle of view θ of 180 degrees or more is realized by mounting the fish eye lens, the extending direction of the intestine turned at an acute angle can be easily checked so that a force may be applied in a correct direction, thereby avoiding such enterobrosia.

Particularly, if the projection 110 has a dome structure, it is preferable that the light part 140 is disposed in a convex surface of the projection 110 having the dome structure. In case of the fish eye lens, if light is incident directly on the fish eye lens, a flare phenomenon occurs. Thus, an illumination angle α or illumination direction of the light part 140 is required to be properly designed such that light from the light part 140 is not incident directly on the fish eye lens. However, it may not be preferable to limit the illumination angle α or illumination direction of the light part 140 in view of the purpose of the observation, and the flare phenomenon may not be sufficiently avoided only by such adjustment. According to the embodiment of the present disclosure, since the light part 140 is disposed in the convex surface of the projection 110 as shown in FIG. 4, a possibility that the light from the light part 140 will be incident directly on the fish eye lens is reduced. Accordingly, there is an advantage of prevention of the flare phenomenon.

Moreover, although not described herein, in a case where the projection 110 has a conical structure, it is preferable that a blocking wall is installed between the fish eye lens and the light part 140 so that the light from the light part 140 is not incident directly on the fish eye lens.

In addition, since the filming device according to the embodiment of the present disclosure can observe the lateral side and rear side of the endoscope, it is preferable to install an additional light part in a side surface of the front end part 100.

While the present disclosure has been described herein in connection with in some embodiments, it should be noted that various changes and modifications may be made without departing from the spirit and scope of the disclosure which can be understood by those skilled in the art. In addition, it should be considered that such changes and modifications fall within the scope of the claims appended herein. Although the present disclosure has been primarily described by way of example in connection with the endoscope for the large intestine, it is not limited thereto but can be applied to other medical endoscopes such as a gastroscope, a cystoscope, a bronchoscope, a pharyngiolaryngoscope, a nasoscope, an otoscope, a laparoscope and the like.

### DESCRIPTION OF REFERENCE SYMBOLS

100: Front end part, 200: Bending part, 300: Insertion part, 400: Manipulation part, 500: Universal cord

## Claims

1. An endoscope, comprising:
a front end part;
a bending part connected to the front end part and including a plurality of bending elements;
an insertion part; and
a manipulation part,
wherein the insertion part is a part connecting the bending part and the manipulation part and moves the front end part and the being part in a coelom as at least a portion of the insertion part is inserted into or withdrawn from the coelom,
the front end part has a projection protruding forward and tapered to have a decreasing width toward its tip,
at least one channel opening is provided in a surface of the projection,
at least one light part is provided in a surface of the projection, and
a filming device having a fish eye lens is installed at the tip of the projection.

2. The endoscope of Claim 1, wherein a cross section of the projection taken in a direction perpendicular to a longitudinal direction of the endoscope is a circle in shape.

3. The endoscope of Claim 2, wherein the projection is dome-shaped.

4. The endoscope of Claim 2, wherein the projection is cone-shaped.

5. The endoscope of Claim 2, wherein a longitudinal axis of the endoscope passes through a center of the circle.

6. The endoscope of Claim 3, wherein the at least one light part is provided in a convex surface of the projection.
